(19) Europäisches Patentamt
European Patent Office
Office européen des brevets

(11) Numéro de publication: **0 031 741**
**A1**

(12) # DEMANDE DE BREVET EUROPEEN

(21) Numéro de dépôt: **80401728.3**

(22) Date de dépôt: **03.12.80**

(51) Int. Cl.³: **C 07 D 217/26**, C 07 D 209/42, C 07 D 209/44, C 07 D 471/04, C 07 D 495/04, A 61 K 31/40, A 61 K 31/47 // (C07D471/04, 235/00, 221/00),(C07D471/04, 221/00, 209/00),(C07D495/04, 333/00, 221/00)

(30) Priorité: **07.12.79 FR 7930046**
**31.07.80 FR 8016875**

(43) Date de publication de la demande: **08.07.81 Bulletin 81/27**

(84) Etats contractants désignés: **BE CH DE GB IT LI NL**

(71) Demandeur: **SCIENCE UNION ET Cie SOCIETE FRANCAISE DE RECHERCHE MEDICALE, 14 rue du Val d'Or, F-92150 Suresnes (FR)**

(72) Inventeur: **Vincent, Michel, 8, allée du Prunier Hardy, F-92220 Bagneux (FR)**
Inventeur: **Remond, Georges, 9, avenue des Etats-Unis, F-78000 Versailles (FR)**
Inventeur: **Laubie, Michel, 35, avenue Foch, F-92420 Vaucresson (FR)**

(74) Mandataire: **Faloux, André, Dr., SCIENCE UNION ET CIE SOCIETE FRANCAISE DE RECHERCHE MEDICALE Service Brevets 22, rue Garnier, F-92200 Neuilly s/Seine (FR)**

(54) **Nouveaux iminoacides substitués, leurs procédés de préparation et leur emploi comme inhibiteurs d'enzymes.**

(57) Les imino acides et leurs dérivés, de formule

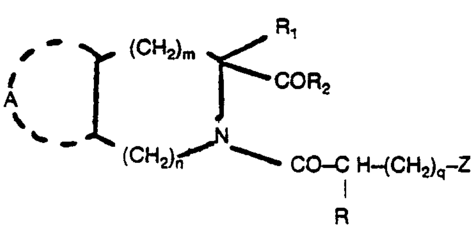

dans laquelle A représente une structure cyclique, mono ou bicyclique, saturée ou non saturée, pouvant incorporer un ou plusieurs hétéroatomes, non substitués ou substitués par un ou plusieurs substituants,

m, n et q sont zéro ou un nombre entier de 1 ou 2,

~~R est de l'hydrogène, un radical méthyle ou benzyle,~~

$R_1$ est de l'hydrogène, un radical alcoyle inférieur jusqu'à 5 atomes de carbone.

$R_2$ est un hydroxyle ou un alcoxy inférieur

Z est un radial carboxy, cyano, hydroxyle, mercapto ou amino, mais dans le cas où Z est mercapto, A ne peut pas être benzo, leurs isomères optiques et de position, leurs sels d'addition et leur procédé de préparation.

Ces composés ont des propriétés thérapeutiques, notamment antihypertensives et cardiovasculaires et peuvent être utilisés comme médicaments.

**EP 0 031 741 A1**

La présente invention se rapporte à de nouveaux imino acides bicycliques et à leurs procédés de préparation.

Plus particulièrement elle concerne des acides Azabicycloalcane 2 - carboxyliques de formule générale I :

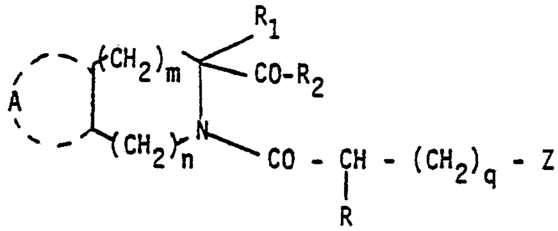

(I)

dans laquelle A représente une structure cyclique, mono ou bicyclique, saturée ou insaturée, pouvant incorporer un ou plusieurs hétéroatomes, non substituée ou substituée par un ou plusieurs substituants.

m, n et q sont zéro ou un nombre entier de 1 ou 2

R est de l'hydrogène, un radical méthyle ou benzyle

$R_1$ est de l'hydrogène, un radical alcoyle inférieur jusqu'à 5 atomes de carbone

$R_2$ est un hydroxyle ou un alcoxy inférieur,

Z est un radical carboxy, cyano, hydroxyle, mercapto ou amino, mais dans le cas où Z est mercapto, A ne peut pas être benzo.

L'invention se rapporte également aux sels de composés de formule générale I. Les composés où $R_2$ est un hydroxyle, peuvent être salifiés par une base minérale ou organique et ceux où Z est amino par un acide minéral ou organique de préférence thérapeutiquement compatible.

Les composés de formule I comportent au moins 2 atomes de carbone asymétriques. Selon la position des substituants et le degré d'hydrogénation il existe de deux à sept centres d'asymétrie.

Les composés racémiques peuvent être dédoublés en leurs mélanges de diastéréoisomères ou d'épimères, ou dédoublés en leurs énantiomères.

Les composés selon l'invention ainsi que leurs sels sont doués de propriétés pharmacologiques intéressantes. Ils inhibent notamment la transformation du décapeptide Angiotensine I en l'octapeptide Angiotensine II par une inhibition de l'enzyme responsable de cette conversion.

Les composés selon l'invention ont une action inhibitrice sur les enzymes comme les carboxypolypeptidases, responsables de la transformation de l'Angiotensine I en Angiotensine II ou sur les enkephalinases. Leur emploi en thérapeutique permet donc de réduire ou même de supprimer l'action d'enzymes en agissant sur un des mécanismes directement responsables de l'hypertension artérielle ou de l'insuffisance cardiaque.

L'invention se rapporte donc à l'emploi en thérapeutique des composés de formule générale I et de leurs sels, notamment pour le traitement de l'hypertension artérielle et l'insuffisance cardiaque.

L'invention s'étend aussi aux compositions pharmaceutiques renfermant comme principe actif au moins un composé de formule générale I ou un de ses sels d'addition, avec une base ou un acide minéral ou organique, en association avec un excipient inerte non toxique pharmaceutiquement acceptable.

En vue de l'emploi en thérapeutique, les composés de formule générale I ou leurs sels sont présentés sous des formes pharmaceutiques convenant pour l'administration par voie parentérale, buccale, perlinguale ou rectale. Les compositions pharmaceutiques selon l'invention renferment outre le principe actif, un ou plusieurs excipients inertes, non toxiques convenant pour l'usage pharmaceutique et/ou un agent liant, un agent aromatisant, un agent de délitement, un agent édulcorant, un agent lubrifiant ou bien encore un véhicule liquide ou solide adapté à l'administration par voie parentérale ou rectale.

Les compositions pharmaceutiques selon l'invention peuvent en outre contenir un autre principe actif d'action complémentaire ou synergistique.

Parmi ces derniers principes actifs, on pourra citer un diurétique et, notamment, un salurétique des substances α-adrénolytiques, β-bloqueurs, antagonistes calciques ou agonistes des récepteurs dopaminergiques.

La posologie utile peut varier largement en fonction de l'âge, du poids du patient, de la sévérité de l'indication thérapeutique ainsi que la voie d'administration. La voie d'administration préférée est la voie buccale mais la voie rectale ou parentérale sont également parfaitement appropriées. D'une manière générale, la posologie unitaire s'échelonnera entre 25 et 250 mg et la posologie journalière entre 100 et 500 mg.

L'invention comprend également un procédé d'obtention des composés de formule générale I, selon lequel on soumet un ester d'alcoyle d'acide azabicyloalkane 2-carboxylique de formule générale II :

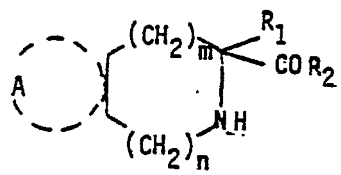

(II)

dans laquelle la définition des substituants A, $R_1$, m et n demeure celle mentionnée précédemment

et $R_2$ représente un radical alcoxy inférieur, à l'action d'un acide substitué de formule générale III :

$$Z' - (CH_2)q - \underset{\substack{| \\ R}}{CH} - COOH \qquad (III)$$

dans laquelle la définition de R et q demeure cèlle fournie précédemment,

Z' représente un radical aminé primaire protégé par les radicaux habituels tels que par exemple le benzyloxycarbonyl, le terbutoxycarbonyl, un radical hydroxylé ou thiol protégé par les radicaux habituels tels que par exemple acyl ou aroyle, ou un radical carboxylique sous forme d'ester, par exemple, ester éthylique,

ou un des dérivés fonctionnels de (III) pour obtenir une amide de formule générale IV :

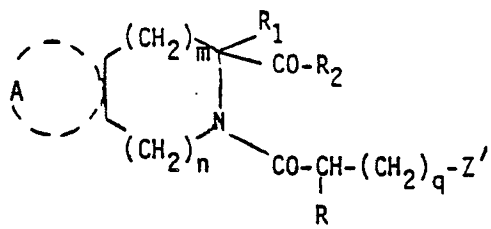

(IV)

dans laquelle Z' possède les définitions fournies précédemment,

$R_2$ représente un radical alcoxy inférieur et les substituants R, $R_1$, A, m, n et q gardent les significations fournies antérieurement, qu'on soumet aux procédés de déprotection habituels, tels que par exemple saponification totale ou partielle et/ou hydrogénolyse, et est ainsi transformée en composé de formule générale (I).

Les composés de formule générale (I) pour lesquels $R_2$ est un hydroxyle donnent des sels avec les bases minérales ou organiques comme par exemple les sels de sodium, de potassium, de lithium, d'ammonium, de calcium, de magnésium, d'aluminium;

Les exemples suivants illustrent l'invention sans toutefois la limiter :

Exemple 1.

2-[3-mercapto 2 (RS) méthyl propionyl] 3-(S) Carbethoxy cis. perhydro isoquinoléine

3,1 g (0,0125 mol) de chlorhydrate de carbethoxy-3 perhydroisoquinoléine préparé selon R.T. RAPALA et al. J. Amer. Chem. Soc. 79, 3770-3772 (1957), 1,75 ml (0,0125 mol) de triéthylamine et 2 g (0,0125 mol) d'acide 2-(RS) Méthyl 3-acétylthio propionique, en solution dans 60 ml de chlorure de méthylène et refroidis à + 5°C sont additionnés de 1,84 g (0,0125 mol) d'hydroxybenztriazole en solution dans 50 ml de chlorure de méthylène, puis de 2,56 g (0,0125 mol) de dicyclohexyl carbodiimide. Après 15 heures d'agitation à température ambiante, le précipité de dicyclohexylurée formé est filtré et le filtrat évaporé à sec sous vide de la trompe à eau, est

redissous dans 100 ml d'acétate d'éthyle, filtré à nouveau et lavé successivement par 50 ml de solution aqueuse saturée de NaCl, 2 x 25 ml de solution aqueuse d'acide citrique à 10 %, 50 ml de NaCl saturé, 2 x 25 ml de solution aqueuse saturée de Na $HCO_3$, enfin par NaCl saturé jusqu'à neutralité. La phase organique est séchée sur $SO_4Ca$, filtrée et le filtrat concentré à sec. Le résidu est chromatographié sur 200 g de Silice en diluant par Acétate d'éthyle/benzène/20/80. 1,85 g (42 %) de 2-/3-acétylthio 2-(RS) méthylpropionyl/3-(RS) carbethoxy cis perhydro isoquinoléine, sont obtenus.

Analyse : $C_{18}$ $H_{29}$ $NO_4$ S

| | C | H | N | S |
|---|---|---|---|---|
| Calculé % | 60,82 | 8,22 | 3,94 | 9,02 |
| Trouvé | 60,94 | 8,11 | 3,92 | 8,68 |

1,7 g de dérivé précédent sont saponifiés à température ambiante 84 heures en solution dans 15 ml de Potasse Normale, 15 ml d'eau et le minimum (15 ml) d'éthanol pour obtenir une solution limpide. Après évaporation sous vide de la majorité de l'éthanol à température ambiante, la solution aqueuse est extraite à l'éther, acidifiée par 15 ml d'HCl Normale, extraite à l'éther de nouveau. La dernière phase éthérée est séchée sur $SO_4Ca$, filtrée et concentrée à sec.

1,1 g (80 %) de 2-/3-mercapto 2-(RS) méthylpropionyl/3-(S) carbethoxy cis. perhydro isoquinoléine, sont obtenus.

Analyse : $C_{14}$ $H_{23}$ $BO_3$ S

| | C | H | N | S |
|---|---|---|---|---|
| Calculé % | 58,92 | 8,12 | 4,92 | 11,23 |
| Trouvé % | 58,45 | 8,26 | 4,61 | 10,97 |

Exemple 2

5(3-mercapto 2-(RS)-méthylpropionyl) 6-(RS) Carboxy 4, 5, 6, 7 tetra hydro [3 H] imidazo [4,5 - C] pyridine

En utilisant le même mode opératoire qu'à l'exemple 1, au départ de l'ester éthylique de 6-(RS) carboxy 4, 5, 6, 7 têtrahydro [3 H] imidazo [4,5-C] Pyridine préparée selon la méthode décrite par Achermann H.S. S.f. Phys. Chem. 284 (1949) 131, et de l'acide 3-acétylthio 2-méthyl propionique, on obtient successivement :

la 5-(3-acéthylthio 2 (RS)-méthyl propionyl) 6-(RS) Carbéthoxy 4, 5, 6, 7 têtrahydro[3 H] imidazo [4,5-C] Pyridine

la 5-(3-thio 2 (RS)-méthylpropionyl) 6-(RS) Carboxy 4, 5, 6, 7, têtrahydro [3 H] imidazo [4,5-C] pyridine

Analyse : $C_{10} H_{12} N_3 O_3 S$

| | C | H | N | S |
|---|---|---|---|---|
| Calculé % | 47,23 | 4,76 | 16,52 | 12,61 |
| Trouvé % | 47,42 | 4,68 | 16,73 | 12,38 |

Exemple 3

Acide 2-[(3-mercapto 2-(RS)méthylpropionyl) 1, 2, 3, 4 têtrahydro [9H]pyrido (3, 4b) indole] 3-(S) carboxylique

En utilisant le mode opératoire de l'exemple 1 au départ de l'ester méthylique de l'acide (1,2,3,4-têtrahydro [9H] pyrido (3,4-b) indole) 3-(S) carboxylique, obtenu selon la méthode décrite par A. Brossi J. Med. Chem. 16 (1973) 419 et de l'acide 3-acétylthio 2-(RS) méthyl propionique, on obtient successivement :

le 2-/3-acétylthio 2-(RS) méthyl propionyl/ 1,2,3,4-tetrahydro 9H pyrido (3,4-b) indole 3-(S) carboxylate de méthyle.

L'acide 2-/(3-mercapto 2-(RS) méthylpropionyl) 1,2,3,4-tétrahydro/9H/pyrido (3,4-b) indole 3-(S)/carboxylique.

Le sel de dicyclohexylamine de l'acide 2-/(3-mercapto 2(RS)-méthyl propionyl) 1,2,3,4-tetrahydro /9H/ pyrido (3,4-b) indole/ 3-(S) carboxylique

Analyse : $C_{16} H_{18} N_2 O_3 S$

| | C | H | N | S |
|---|---|---|---|---|
| Calculé % | 60,36 | 5,70 | 8,80 | 10,07 |
| Trouvé % | 60,20 | 5,59 | 8,83 | 9,81 |

Exemple 4

Acide 2-(3-cyano 2-(RS)-méthylpropionyl) 1,2,3,4-tetrahydro isoquinoléine 3-(S) carboxylique

En opérant selon le mode opératoire de l'exemple 1 au départ de l'ester méthylique de l'acide 1,2,3,4-tétrahydro-isoquinoléine 3(S)-carboxylique synthétisé selon S. ARCHER J. Org. Chem. 16 431 (1951) et de l'acide 3-cyano 2-(RS) méthylpropionique, on obtient l'ester méthylique de l'acide 2-(3-cyano 2-(RS) méthylpropionyl) 1,2,3,4-tétrahydroisoquinoléine 3(S)-carboxylique.

L'ester méthylique est saponifié par agitation avec une solution de Baryte à 20° C. On sépare le précipité de carbonate de Baryum par filtration, le lave à l'eau, réunit les phases aqueuses et les concentre à sec sous vide.

Le résidu sec est recristallisé de l'acétate d'éthyle.

L'acide 1,2,3,4-tétrahydro 2-/3-cyano 2-(RS)méthyl propionyl/ isoquinoléine 3-(S) carboxylique est obtenu sous forme cristalline.

Analyse $C_{14}H_{14}N_2O_3$

| | C | H | N |
|---|---|---|---|
| Calculé % | 65,10 | 5,46 | 10,85 |
| Trouvé % | 64,73 | 5,74 | 10,80 |

Exemple 5

N-/3-mercapto 2-(RS) méthyl propionyl7 2-(RS) carboxy perhydroindole

En opérant selon le procédé décrit dans l'exemple 1 à partir d'acide 3-acétylthio 2-(RS) méthyl propionique et de 2-(RS) carbéthoxy perhydroindole préparé par réduction de 2-(RS) carbéthoxy indoline selon le procédé décrit dans l'exemple 13, on obtient successivement :

- le N-/3-acétylthio 2-(RS) méthyl propionyl7 2-(RS) carbéthoxy perhydroindole

- le N-/3-mercapto 2-(RS) méthyl propionyl7 2-(RS) carboxy perhydroindole.

Analyses : $C_{13}H_{21}NO_3S$

| | C | H | N | S |
|---|---|---|---|---|
| Calculé % | 57,54 | 7,80 | 5,16 | 11,81 |
| Trouvé % | 57,31 | 7,79 | 4,86 | 11,64 |

Exemple 6

N/3-mercapto 2-(RS) méthyl propionyl7 5-(RS) carboxy 4,5,6,7-tétrahydro thiéno (3,2-C) pyridine

En opérant selon le procédé décrit dans l'exemple 1 à partir d'acide 3-acétylthio 2-(RS) méthyl propionique et de 6-(RS) méthoxy 4,5,6,7

-tétrahydro (3,2-C) pyridine préparée selon le brevet français N° 7700408, on obtient successivement :

- la N/3-acétylthio 2 -(RS) méthyl propionyl/6-(RS) méthoxy 4,5,6,7-tétrahydro (3,2-C) pyridine

- la N/3-mercapto 2-(RS) méthyl propionyl/ 6-(RS) carboxy 4,5,6,7 tétrahydro thiéno (3,2-C) pyridine

Analyses : $C_{12}H_{15}NO_3S_2$

| | C | H | N | S |
|---|---|---|---|---|
| Calculé % | 50,50 | 5,30 | 4,91 | 22,47 |
| Trouvé % | 50,42 | 5,16 | 4,63 | 22,18 |

Exemple 7

N-/3-mercapto 2-(RS) méthyl propionyl/ 1-(RS) carboxy perhydroisoindole

En opérant selon le procédé décrit dans l'exemple 1 à partir d'acide 3-acétylthio 2-(RS) méthyl propionique et de 1 -(RS) carbométhoxy perhydroiso indole préparé par réduction de 1 -(RS) carbéthoxy isoindoline selon le procédé décrit dans l'exemple 13 pour la 2-(RS) carbéthoxyindoline, on obtient successivement :

- le N/3-acéthylthio 2-(RS) méthyl propionyl/ 1-(RS) carbéthoxy perhydroisoindole.

- le N/3-mercapto 2- (RS) méthyl própionyl/ 1-(RS) carboxy perhydroisoindole.

Analyses : $C_{13}H_{21}NO_3S$

par RMN, présence des signaux caractéristiques :

| | | |
|---|---|---|
| δ | 1 H | 4,5 ppm (d) |
| δ | 1 H | 3,6 ppm (m) |
| δ | 2 H | 2,8 ppm (d) |
| δ | 2 H | 2,4 ppm |
| δ | 10 H | 1,5 ppm (m) |
| δ | 3 H | 1,2 ppm (d) |
| δ | 2 H | 8 ppm échangeables par $D_2O$. |

Exemple 8

1 -/3-mercapto 2- (RS)méthyl propionyl/2-(RS) carboxy perhydroquinoléïne

Ce composé est obtenu en saponifiant par la soude normale hydroalcoolique la 1-/3-acétylthio 2-(RS) méthyl propionyl/2-(RS) carboxy perhydroquinoléïne elle-même obtenue en opérant dans le chlorure de méthylène, en présence de N-diméthylaniline à partir de chlorure de l'acide 3-acétylthio 2-(RS) méthyl propionique et de 2-(RS) carboxy perhydroquinoléïne préparée par réduction, selon le procédé de l'exemple 13, de la 2-(RS) carboxy 1,2,3,4 tétrahydroquinoléïne, elle-même préparée selon H. WIELAND et al. (Ber. 61 B, 2371-2381 (1928).

Analyses $C_{14} H_{23} NO_3 S$

| | C | H | N | S |
|---|---|---|---|---|
| Calculé % | 57,11 | 8,22 | 4,76 | 10,89 |
| Trouvé % | 57,27 | 7,97 | 4,20 | 10,97 |

Exemple 9

2-/3-carboxy 2-(RS) méthyl propionyl/ 3-(S) carboxy 1,2,3,4 tétrahydro-isoquinoléïne

En opérant selon le procédé de l'exemple 8, à partir du chlorure de l'acide 3-carbométhoxy 2-(RS)méthyl propionique et de 3-(RS) carbométhoxy 1,2,3,4 tétrahydro isoquinoléïne, on obtient successivement :

- la 2-/3 - carbométhoxy 2-(RS) méthyl propionyl/ 3-(S) carbométhoxy 1,2,3,4 tétrahydroisoquinoléïne

- la 2/3-carboxy 2-(RS) méthyl propionyl/3-(S) carboxy 1,2,3,4 tétrahydro isoquinoléïne

Analyses $C_{15}H_{17}NO_5$

| | C | H | N |
|---|---|---|---|
| Calculé % | 61,85 | 5,88 | 4,81 |
| Trouvé % | 61,92 | 5,93 | 4,65 |

Exemple 10

2-/(S) alanyl/ 3-(S) carboxy 1,2,3,4-tétrahydroisoquinoléïne

6,01 g (0,0264 mol) de chlorhydrate de l'ester méthylique de l'acide 1,2,3,4 tétrahydro isoquinoléïne 3-(S) carboxylique sont dissous dans 50 ml d'eau et la solution alcalinisée à pH 11 par $NH_4OH$ puis extraite par deux fois 50 ml d'éther sulfurique. Les solutions éthérées réunies sont séchées sur sulfate de calcium, filtrées et évaporées à sec. L'amino ester résiduel (5,04 g) est dissous dans 30 ml de diméthylformamide et cette solution ajoutée à une solution agitée de 5 g (0,0264 mol) de terbutyloxy carbonyl (S) alanine dans 30 ml de diméthylformamide refroidie à 0, + 5°C. A la solution obtenue sont ajoutées successivement 3,6 g (0,0264 mol) d'hydroxy-1 benztriazole dissous dans 40 ml de diméthylformamide puis 5,45 g (0,0264 mol) de dicyclohexylcarbodiimide dissous dans 30 ml de chloroforme.

La masse réactionnelle est agitée pendant 18 h. en laissant remonter à température ambiante. La dicyclohexylurée formée est filtrée et le filtrat évaporé à sec sous 0,1 mm Hg laisse un résidu qui est redissous

dans 50 ml d'acétate d'éthyle et filtré à nouveau pour séparer un second jet de dicyclohexylurée. Le filtrat est lavé successivement par 80 ml de solution aqueuse saturée de NaCl, 2 x 40 ml de solution aqueuse citrique à 10 %, à nouveau 80 ml de solution aqueuse saturée de NaCl, 2 x 40 ml de solution aqueuse saturée de $CO_3$ HNa, enfin par NaCl en solution aqueuse saturée jusqu'à neutralité.

La phase organique est séchée sur $SO_4$ Ca, filtrée et évaporée à sec sou vide. On obtient 9,1 g (95 %) de 2-[terbutoxy carbonyl (S) alanyl] 3-(S) carbométhoxy 1,2,3,4 tétrahydro isoquinoléïne fondant à 98-100° (banc Kofler).

1,45 g (0,004 mol) de ce composé sont dissous dans 20 ml de méthanol et la solution obtenue additionnée de 4,4 ml de soude aqueuse normale.

La solution est abandonnée 20 heures à température ambiante. Le méthanol est évaporé sous vide de la trompe à eau et le résidu repris par 20 ml d'eau. Après extraction des insaponifiables par l'acétate d'éthyle la phase aqueuse est acidifiée par 4,4 ml de HCl normal. Le précipité formé est extrait par 2 x 20 ml d'acétate d'éthyle qui est séché sur $SO_4$Ca, filtré et évaporé.

On obtient 1,3 g (93 %) de 2-[terbutoxy carbonyl (S) alanyl] 3-(S) carboxy 1,2,3,4 tétrahydro isoquinoléïne.

1,1 g (0,00316 mol) de ce dérivé sont agités à + 5°C avec 4,5 ml d'acide trifluoroacétique à l'abri de l'humidité.

La solution obtenue est concentrée à sec sous 0,1 mmHg. Le résidu cristallin hydroscopique d'évaporation est le produit cherché, sous forme de trifluoroacétate.

0,7 g (0,0019 mol) de trifluoroacétate précédent sont transformés en 0,45 g (94 %) d'acide aminé libre correspondant, par passage sur 50 g de résine sulfonée Dowex W x 8 $H^+$ puis élution par 500 ml d'ammoniaque normale et concentration à sec des éluats ammoniacaux sous vide de la trompe à eau.

Point de fusion : 170° C avec décomposition.

Analyse $C_{13} H_{16} N_2 O_3$

| | C | H | N |
|---|---|---|---|
| Calculé % | 62,89 | 6,50 | 11,29 |
| Trouvé % | 62,58 | 6,18 | 11,24 |

Exemple 11

2 - /3-amino-2(RS) méthyl propionyl7 3-(S) carboxy 1,2,3,4-isoquinoléïne

En opérant selon le procédé décrit dans l'exemple 10 à partir d'acide 3-terbutoxycarbonylamino 2-(RS) méthyl propanoïque et de 3 -(RS) carbométhoxy 1,2,3,4 tétrahydro isoquinoléïne, on obtient successivement :

- la 2-/3-terbutoxycarbonylamino 2-(RS) méthyl propionyl7 3-(S) carbo-méthoxy 1,2,3,4, tétrahydro isoquinoléïne.
- la 2-/3-terbutoxycarbonylamino 2-(RS) méthyl propionyl7 3-(S) carboxy 1,2,3,4 tétrahydro isoquinoléïne.
- la 2-/3-amino 2-(RS) méthyl propionyl7 3 -(S) carboxy 1,2,3,4 tétrahydro isoquinoléïne, qui est transformée en chlorhydrate par dissolution dans HCl normal en excès et concentration à sec.

Analyse $C_{14} H_{19} Cl N_2O_3$

| | C | H | N | Cl % |
|---|---|---|---|---|
| Calculé % | 56,28 | 6,41 | 9,38 | 11,87 |
| Trouvé % | 56,44 | 6,59 | 9,04 | 11,94 |

Exemple 12

2-[4-amino 2-(RS)-méthyl butyryl] 3-(S) carboxy 1,2,3,4 tétrahydro isoquinoléïne

En opérant selon le procédé décrit dans l'exemple 10 à partir d'acide 4 -terbutoxycarbonylamino 2-(RS) méthyl butyrique et de 3-(S) carbométhoxy 1,2,3,4 tétrahydro isoquinoléïne, on obtient successivement :

- la 2-[4-terbutoxycarbonylamino 2-(RS) méthyl butyryl] 3-(S) carbométhoxy 1,2,3,4, tétrahydro isoquinoléïne

- la 2-[4-terbutoxycarbonylamino 2-(RS) méthyl butyryl]3 - (S) carboxy 1,2,3,4 tétrahydro isoquinoléïne.

- la 2 -[4-amino 2-(RS) méthyl butyryl] 3-(S) carboxy 1,2,3,4, tétrahydro isoquinoléïne qui est transformée en chlorhydrate selon la méthode décrite dans l'exemple 11.

Analyse $C_{15} H_{21} Cl N_2 O_3$

| | C | H | N | Cl % |
|---|---|---|---|---|
| Calculé % | 57,60 | 6,77 | 8,96 | 11,33 |
| Trouvé % | 57,31 | 6,34 | 8,69 | 10,69 |

Exemple 13

1 -(S) alanyl 2-(S) carboxy perhydroindole

En opérant selon le procédé décrit dans l'exemple 10 à partir de terbutoxycarbonyl (S) alamine et de 2-(S) carbéthoxy perhydroindole, on obtient successivement :

- le 1-[terbutoxycarbonyl (S) alanyl]2 - (S) carbêthoxy perhydroindole

- le 1-[terbutoxycarbonyl (S) alanyl]2 - (S) carboxy perhydroindole

- le 1-(S) alanyl 2 - (S) carboxy perhydroindole

Analyse $C_{12} H_{20} N_2O_3$

| | C | H | N |
|---|---|---|---|
| Calculé % | 59,98 | 8,39 | 11,10 |
| Trouvé % | 59,53 | 8,24 | 11,43 |

Le 2(S) carbethoxy perhydroindole intermédiaire est préparé par le procédé suivant :

31,5 g (86 %) de 2-(RS) carboxy indoline sont obtenus par sopinification dans 250 ml de soude normale et 150 ml d'éthanol pendant 18 h., à la température ambiante, de 43 g (0,224 mol) d'ester éthylique correspondant préparé selon E.J. COREY et al. (J. Amer. Chem. Soc. 1970, 92 p. 2476).

La solution hydroalcoolique est concentrée au 1/2, neutralisée par 25 ml d'acide chlorhydrique 10 N, filtrée, lavée à l'eau et séchée.

L'acide brut est purifié par passage sur une colonne de résine échangeuse d'ion Dowex 50 W x 8 $H^+$ et élution par l'ammoniaque aqueuse 2 N. Le sel d'ammonium obtenu est dissous dans le minimum d'eau et l'acide précipité pour la quantité théorique d'HCl. Il est essoré, lavé à l'eau et séché à l'air.

Analyse (du sel d'ammonium) $C_9 H_{12} N_2 O_2$

| | C | H | N |
|---|---|---|---|
| Calculé % | 59,99 | 6,71 | 15,54 |
| Trouvé % | 59,93 | 6,71 | 15,29 |

60,5 g (0,37 mol) de 2-(RS) carboxy indoline préparé au stade précédent sont ajoutés à une solution de 44,9 g (0,37 mol) de (+) α méthyl benzylamine dans 400 ml d'éthanol anhydre. Le précipité obtenu est essoré et digéré dans 350 ml d'isopropanol anhydre au reflux. Après refroidissement la suspension est filtrée, le précipité est lavé par un peu d'isopropanol et séché.

Poids obtenu de 2-(S) carboxy indoline, sel de (+) méthyl benzylamine : 29,8 g.

$$\alpha_D^{21} = + 5,3° \text{ (C = 1 \%, éthanol)}$$

La 2-(S) carboxy indoline est préparée avec un rendement théorique par dissolution de 10 g du sel précédent (0,029 mol) dans 50 ml d'eau et acidification par 29 ml d'acide chlorhydrique normal.

Le précipité est essoré, lavé à l'eau, distillé et séché. Pureté optique : 96 % (C.P.V. après dérivation sous forme d'amide de l'acide (-) camphanique).

La 2 -(R) carboxy indoline a été obtenue par le même procédé à partir de 2 (RS) carboxy indoline et de (-) α méthyl benzylamine.

Les configurations absolues des acides (S) et (R) ont été déterminées comme suit :

- des quantités analytiques (environ 0,5 g) de chacun des acides sont transformés en esters éthyliques par traitement par le chlorure de thionyle et l'éthanol.

- les esters sont réduits par l'"hydrure de lithium aluminium" selon E.J. COREY (Lot. Cit.) en alcools primaires correspondants, qui sont identifiés par leur pouvoir rotatoire aux alcools décrit par E.J. COREY (loc. cit.), dont les configurations absolues respectives sont connues.

11 g de 2-(S) carboxy -2 indoline, sel de (+) α méthyl benzylamine (0,032 mol) préparé au stade précédent sont dissous dans 100 ml d'eau et transformés en acide correspondant par addition de 32 ml d'HCl N. L'acide est essoré, lavé à l'eau et séché en dessicateur sur anhydride phosphorique, puis mis en suspension dans 50 ml d'éthanol anhydre. A 0, + 5°, 3,9 ml de chlorure de thionyle sont ajoutés en 10 minutes sous agitation et l'agitation est maintenue 1 heure à 25°C, puis 1 heure à 50° C.

Le mélange est abandonné la nuit à 25°, puis concentré à sec sous vide de la trompe à eau à 40° et repris par 50 ml de benzène anhydre puis amené de nouveau à sec, mis en suspension dans l'éther sulfurique anhydre et essoré.

Le chlorhydrate de 2-(S) carbéthoxy indoline obtenu est hydrogéné en solution dans 150 ml d'eau en présence de 2 g de charbon palladié pendant 8 heures à 45° C sous 50 kg/$cm^2$.

Après refroidissement et filtration du catalyseur, le filtrat est évaporé à sec. Le résidu est le produit cherché sous forme de chlorhydrate.

Poids : 6,9 g (93 %)

Analyse $C_{11}H_{20}ClNO_2$

| | C | H | N | Cl |
|---|---|---|---|---|
| Calculé % | 56,52 | 8,62 | 5,99 | 15,17 |
| Trouvé % | 55,52 | 8,53 | 5,96 | 15,16 |

Exemple 14

Etude pharmacologique des composés de l'invention.

Les composés selon l'invention sont testés par administration à des lots de chiens de race Mongrel, non anesthésiés, maintenus en respiration forcée à l'aide d'un appareil respiratoire Mark VII Bird.

La pression artérielle des chiens a été mesurée par un capteur de pression Statham P23Db après cathétérisation de l'aorte par l'intermédiaire de l'artère fémorale. L'enregistrement est réalisé par un appareil enregistreur Brush 400.

L'angiotensine I et l'angiotensine II sont injectées aux animaux par voie intra-veineuse à la dose de 0,3 γ/Kg. On établit une courbe dose/activité pour chacune de ces hormones. On administre ensuite les composés selon l'invention par voie intraveineuse. On établit ensuite une deuxième courbe dose/activité pour l'angiotensine I et pour l'angiotensine II après administration des produits essayés.

Les résultats obtenus sont réunis dans le tableau suivant. Les composés décrits dans l'invention contiennent plusieurs carbones asymétriques et peuvent être dédoublés en leurs énantiomères dont un seul recèle l'activité mesurée. On a indiqué entre parenthèses, la dose d'énantiomère actif contenu dans chacune des doses de composé testé.

POURCENTAGES D'INHIBITION

| Composé de l'exemple N° | Dose mg/Kg/IV | TEMPS 15 minutes | 30 minutes | 1 heure | 1 h 30 min. | 2 heures | 3 heures |
|---|---|---|---|---|---|---|---|
| 1 | 20 (10) | 100 | 100 | 90 | 76 | 70 | 42 |
| 5 | 10 (2,5) | 100 | 92 | 92 | 92 | 92 | 86 |
| | 5 (1,25) | 100 | 100 | 100 | 90 | 71 | 54 |
| 6 | 20 (5) | 47 | 51 | 37 | 30 | 26 | 23 |
| 7 | 10 (2,5) | 83 | 64 | 62 | 48 | 40 | 26 |
| 8 | 10 (2,5) | 72 | 69 | 64 | 50 | 36 | 31 |
| 9 | 20 (10) | 38 | 35 | 25 | 25 | 48 | 25 |

Exemple 15

Solution injectable pour perfusion veineuse

| | |
|---|---|
| N-[3-mercapto 2-(RS) méthyl propionyl]2-(RS) carboxy perhydro-indole . . . . . . . . . . . . . . . . . . . . . . . . | 0,015 g |
| perhydroxybenzoate de méthyle . . . . . . . . . . . . . . | 0,15 |
| perhydroxybenzoate de propyle . . . . . . . . . . . . . . | 0,15 |
| chlorure de sodium . . . . . . . . . . . . . . . . . . . . | 8,00 |
| eau distillée q.s.p. . . . . . . . . . . . . . . . . . . . . | 1 000,00 ml |

Exemple 16

| | |
|---|---|
| N-[3-mercapto 2-(RS) méthyl propionyl]2-(RS) carboxy perhydro-indole . . . . . . . . . . . . . . . . . . . . . . . . | 50,00 g |
| amidon de blé . . . . . . . . . . . . . . . . . . . . . . | 100,00 |
| amidon de maïs . . . . . . . . . . . . . . . . . . . . . . | 80,00 |
| caséine formolée . . . . . . . . . . . . . . . . . . . . | 20,00 |
| stéarate de magnésium . . . . . . . . . . . . . . . . . | 15,00 |
| talc . . . . . . . . . . . . . . . . . . . . . . . . . . . . | 20,00 |

pour 1 000 comprimés à 50 mg de principe actif.

REVENDICATIONS

1. Les nouveaux imino acides et leurs dérivés, de formule générale I :

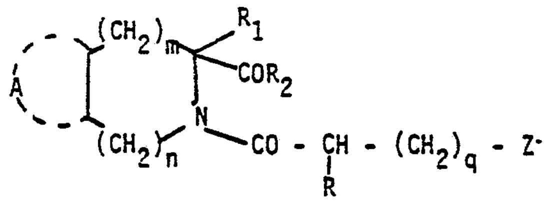

(I)

dans laquelle A représente une structure cyclique, mono ou bicyclique, saturée ou non saturée, pouvant incorporer un ou plusieurs hétéroatomes, non substitués ou substitués par un ou plusieurs substituants,

m, n et q sont zéro ou un nombre entier de 1 ou 2,

R est de l'hydrogène, un radical méthyle ou benzyle,

$R_1$ est de l'hydrogène, un radical alcoyle inférieur jusqu'à 5 atomes de carbone.

$R_2$ est un hydroxyle ou un alcoxy inférieur

Z est un radical carboxy, cyano, hydroxyle, mercapto ou amino, mais dans le cas où Z est mercapto, A ne peut pas être benzo, sous forme racémique ou optiquement active et leurs diastéréoisomères.

2. Les sels d'addition avec une base minérale ou organique des composés selon la revendication 1 pour lesquels $R_2$ est un hydroxyle, ou avec un acide minéral ou organique de ceux où Z est amino.

3. Le N- [3-mercapto-2 (RS) méthyl propionyl] 2-(RS) carboxy perhydroindole, ses énantiomères et leurs sels pharmaceutiquement compatibles.

4. Le 2- [3-mercapto 2- (RS) méthyl propionyl] 3-(S) carbethoxy cis, perhydro isoquinoléine et ses énantiomères.

5. Le N- [3-mercapto 2-(RS) méthylpropionyl] 1-(RS) carboxy perhydro isoindole, ses énantiomères et leurs sels pharmaceutiquement compatibles.

6. Un procédé d'obtention des composés de formule générale I, caractérisé en ce qu'on soumet un ester d'alcoyle d'acide azabicycloalcane α-carboxylique de formule générale II :

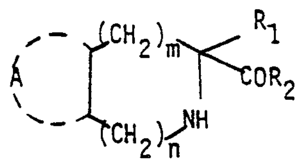

(II)

dans laquelle la définition des substituants A, $R_1$, m et n demeure celle mentionnée ci-dessus

et $R_2$ représente un radical alcoxy inférieur, à l'action d'un acide substitué de formule générale III :

$$Z' - (CH_2)_q - \underset{R}{CH} - COOH \qquad (III)$$

dans laquelle la définition de R et q demeure celle fournie antérieurement,

Z' représente un radical aminé primaire protégé par les radicaux habituels tels que par exemple le benzyloxycarbonyl, le terbutoxycarbonyl, un radical hydroxylé ou mercapto protégé par les radicaux habituels tels que par exemple acyl ou aroyle, ou un radical carboxylique sous forme d'ester

ou un des dérivés fonctionnels de (III), pour obtenir une amide de formule générale IV :

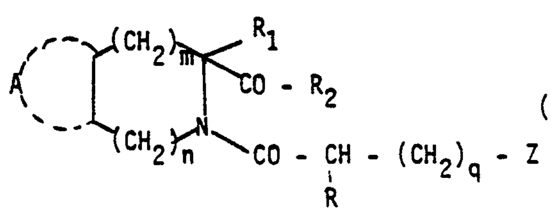

(IV)

dans laquelle Z' possède les définitions fournies précédemment,

$R_2$ représente un radical alcoxy inférieur et les substituants R, $R_1$, A, m, n et q gardent les significations fournies antérieurement, et

qu'on soumet aux procédés de déprotection habituels tels que par exemple saponification totale ou partielle et/ou hydrogénolyse, et est ainsi transformée en composé de formule générale (I).

7. Les compositions pharmaceutiques renfermant à titre de principe actif au moins un composé selon les revendications 1. ou 2. en association avec un excipient ou un véhicule inerte non toxique thérapeutiquement compatible.

8. Une composition pharmaceutique selon la revendication 7. dans laquelle l'excipient ou le véhicule sont ceux qui conviennent pour l'administration par voie parentérale, buccale, rectale ou sublinguale.

9. Une composition pharmaceutique selon la revendication 7. caractérise en ce qu'elle renferme un autre principe actif d'action complémentaire ou synergistique.

10. Une composition pharmaceutique selon les revendications 7. et 9. renfermant le principe actif à la dose de 25 à 250 mg, utilisable dans le traitement de l'hypertension artérielle et de l'insuffisance cardiaque.

Office européen des brevets

# RAPPORT DE RECHERCHE EUROPEENNE

Numéro de la demande 0031741

EP 80 40 1728

| DOCUMENTS CONSIDERES COMME PERTINENTS | | | CLASSEMENT DE LA DEMANDE (Int. Cl. 3) |
|---|---|---|---|
| Catégorie | Citation du document avec indication, en cas de besoin, des parties pertinentes | Revendication concernée | |
| | GB - A - 1 524 481 (SOC. FARMACEUTICI ITALIA)<br>* Pages 1,2 *<br>-- | 1 | C 07 D 217/26<br>209/42<br>209/44<br>471/04<br>495/04<br>A 61 K 31/40<br>31/47//<br>C 07 D 471/04<br>235/00<br>221/00)<br>C 07 D 471/04<br>221/00<br>209/00)<br>C 07 D 495/04 |
| P | EP - A - 0 012 845 (TANABE SEIYAKU CO., LTD.)<br>* Revendications *<br>-- | 1-5 | |
| P | EP - A - 0 018 104 (TAKEDA YAKUHIN)<br>* Revendications *<br>-- | 1-5 | DOMAINES TECHNIQUES RECHERCHES (Int. Cl. 3)<br>C 07 D 217/26<br>471/04<br>209/42<br>209/44<br>495/04 |
| P | FR - A - 2 448 533 (MORTON-NORWICH PRODUCTS)<br>* Revendications *<br>-- | 1-5 | |
| A | GB - A - 975 835 (J.R. GEIGY)<br>* Pages 1,2 *<br>---- | 1 | CATEGORIE DES DOCUMENTS CITES<br>X: particulièrement pertinent<br>A: arrière-plan technologique<br>O: divulgation non-écrite<br>P: document intercalaire<br>T: théorie ou principe à la base de l'invention<br>E: demande faisant interférence<br>D: document cité dans la demande<br>L: document cité pour d'autres raisons |
| X | Le présent rapport de recherche a été établi pour toutes les revendications | | &: membre de la même famille, document correspondant |

| Lieu de la recherche | Date d'achèvement de la recherche | Examinateur |
|---|---|---|
| La Haye | 22-01-1981 | BRIGHENTI |

Office européen des brevets

**RAPPORT DE RECHERCHE EUROPEENNE**

0031741

Numéro de la demande

EP 80 40 1728

-2-

| DOCUMENTS CONSIDERES COMME PERTINENTS | | | CLASSEMENT DE LA DEMANDE (Int. Cl. 3) |
|---|---|---|---|
| Catégorie | Citation du document avec indication, en cas de besoin, des parties pertinentes | Revendication concernée | |
| | | | C 07 D 333/00<br>221/00) |
| | | | DOMAINES TECHNIQUES RECHERCHES (Int. Cl. 3) |
| | | | |

OEB Form 1503.2 06.78